# EUROPEAN PATENT SPECIFICATION

(11) **EP 0 805 677 B1**
(45) Date of publication and mention of the grant of the patent: **15.06.2005**
(21) Application number: 95944804.4
(22) Date of filing: 26.01.1995
(51) Int. Cl.: A61K 9/48, A61K 9/52, A61K 9/56, A61K 9/36, A61K 31/415, A61K 9/20

(54) **EXTENDED RELEASE CLONIDINE FORMULATION**
CLONIDINHALTIGES ARZNEIMITTEL MIT VERZÖGERTER WIRKSTOFFABGABE
PREPARATION DE CLONIDINE A LIBERATION PROLONGEE

(43) Date of publication of application: 12.11.1997
(73) Proprietor: Horacek, H., Joseph, Charlotte, NC 28227 (US)
(72) Inventor: Horacek, H., Joseph, Charlotte, NC 28227 (US)
(74) Representative: Blake, John Henry Francis
(86) International application number: PCT/US1995/001029
(87) International publication number: WO 1996/022768

(56) References cited:
- US-A- 4 871 548
- DATABASE WPI Week 8714 Derwent Publications Ltd., London, GB; AN 87-099083 [14] XP002055889 & JP 62 048 618 A (ZERIA SHINYAKU KOGYO KK) 3 March 1987
- CHEMICAL ABSTRACTS, vol. 106, no. 26, 29 June 1987 Columbus, Ohio, US; abstract no. 219476, XP002055888 & A.I.TENTSOVA ET AL.: "MECHANISM OF CLOFELIN RELEASE FROM SOLID DISPERSION SYSTEMS WITH ETHYL CELLULOSE" FARMATSIYA, vol. 36, no. 2, - 1987 MOSCOW, pages 16-19,
- PATENT ABSTRACTS OF JAPAN vol. 011, no. 371 (C-462), 3 December 1987 & JP 62 145014 A (TEIJIN LTD.), 29 June 1987,

## Description

### TECHNICAL FIELD

The present invention relates to an extended release formulation of clonidine. Specifically, it relates to an oral dosage form of clonidine which provides a release period suitable for twice or three times daily dosing while exhibiting good bioavailability.

### BACKGROUND ART

Clonidine is a well known and widely used alpha-adrenergic agonist. Clonidine is effective in the treatment of a wide range of clinical disorders including hypertension; prophylaxis of common migraine headaches; subduing motor tics such as in Tourette's syndrome; and decreasing hyperactivity, impulsivity and over excitability in Attention Deficit Hyperactivity Disorder, manic states and many other clinical syndromes which involve over arousal.

Clonidine is given in either an oral dose in tablet form three to four times per day or via a transdermal patch. In the oral formulation currently in use, clonidine is almost completely absorbed from the gastrointestinal tract, but it is subject to rapid liver metabolism. The biological half-life ranges from about four to six hours after oral administration, with wide interpatient variability.

The traditional oral formulations of clonidine have disadvantages. The oral dose has the main side-effect of sedation, particularly about an hour after the given dose when the patient may become transiently sedated, even falling asleep. Because the half-life of this dosage form of clonidine is only about four to six hours, there is also the problem of the drug wearing off with some rebound hyperarousal. This can occur in the middle of the night causing insomnia, and even nightmares in some cases. Such side effects have limited the practical usefulness of oral clonidine. A transdermal patch has partially solved these problems by providing a more stable serum level (U.S. Pat. No. 4,201,211). The transdermal patch, however, has the disadvantage of producing a high rate of contact dermatitis. There are also problems with patch adherence to the skin in humid environments and with active individuals. The patch may need replacement after extended swimming or exertion. The inconvenience of the patch can lead to reduced patient compliance

For the foregoing reasons, there is a need for a clonidine formulation which is capable of stable therapeutic effects by maintaining a constant serum level for an extended period in order to avoid the "peak and trough" side effects of transient sedation at peak serum levels and rebound exacerbation of symptoms at trough levels. The clonidine formulation should be easy and inexpensive to manufacture and convenient for the patient to use.

Cited in the International Search Report is US-4871548-A, which discloses a dosage form comprising a low number average molecular weight hydroxypropylmethyl cellulose, a high number average molecular weight hydroxypropylmethyl cellulose, and a beneficial drug. Clonidine is cited as an example of a beneficial drug.

The low number average molecular weight hydroxypropylmethyl cellulose has a number average molecular weight of from about 9,000 to 30,000. The high number average molecular weight hydroxypropylmethyl cellulose has a number average molecular weight of greater than 30,000 to 350,000. The cellulose ether components operate are indicated to act as a unit in a moving fluid for controlling the rate of release of a beneficial drug from the dosage form.

Among the documents cited in the European Search Report, JP-62-048618-A discloses sustained release granule comprising a medicine and a sustained release material, which is coated with a release controlling material. The release controlling material may comprise various cellulose ethers.

### SUMMARY OF THE INVENTION

The present invention is directed to an extended release clonidine formulation which satisfies these needs.

The present invention provides an oral pharmaceutical dosage unit formulation for the extended release of clonidine to effect central alpha-adrenergic stimulation over a prolonged period upon administration thereof, wherein the oral dosage unit comprises:
a. 0.025 mg to 0.40 mg of a compound of the formula 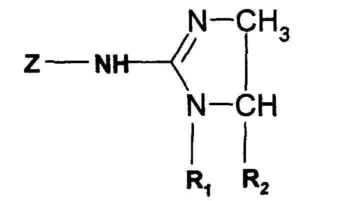 wherein R₁ and R₂ are each selected from the group consisting of hydrogen, methyl and ethyl, and Z is selected from the group consisting of:
   2,6-dichlorophenyl,
   2-trifluoromethyl-phenyl,
   2-chloro-6-methyl-phenyl, 2-methyl-4-chloro-phenyl,
   2-chloro-4-methyl-phenyl,
   2-chloro-4-ethyl-phenyl,
   2-chloro-6-ethyl-phenyl,
   2-chloro-4-tert,butyl-phenyl,
   2,6-dichloro-4-methyl-phenyl,
   2,4-dichloro-6-methyl-phenyl,
   2,4-dimethyl-6-chloro-phenyl, and
   2,6-dimethyl-4-chloro-phenyl,
   and its non-toxic pharmacologically acceptable acid addition salts;
   as an homogenous mixture in
b. 30 to 70 percent by weight of one or more cellulose ethers, all having a number average molecular weight of greater than 30,000;
c. 30 to 70 percent by weight of therapeutically inert, pharmaceutically acceptable and adjunct materials.

### DESCRIPTION OF INVENTION

The present invention has many advantages, particularly the minimization of the side effects of the traditional oral clonidine formulation. As a result of the extended delivery of the drug, the formulation of the present invention is capable of maintaining a constant level of clonidine in the blood thereby avoiding the side effects of transient sedation and rebound hyperarousal and providing more stable therapeutic effects. Twice or three times daily dosages are all that is required. The extended release clonidine formulation of the present invention is also more convenient to use than the transdermal patches thereby resulting in better patient compliance.

Experience has shown that the foundation of all mental health must begin with an adequate night's rest. Attention Deficit Hyperactivity Disorder patients often have a lifetime history of difficulty getting to sleep. This difficulty with state regulation appears to be part of the Attention Deficit Hyperactivity Disorder complex of symptoms. Clonidine has been found to be very useful in aiding the onset of sleep in Attention Deficit Hyperactivity Disorder patients as the sedation experienced approximately one hour after the traditional oral tablet dose of clonidine which is troublesome in the day is often very useful at night in aiding the onset of sleep. One difficulty in a significant number of patients is the problem of waking about four to six hours later, sometimes with a nightmare. The rebound hyperarousal can limit the use of clonidine for this purpose. The extended release clonidine formulation of the present invention overcomes this problem and is a safe, non-habit forming medication effective in regulating sleep and wakefulness which has often proven very valuable in controlling adverse symptoms in the day such as inattention, distractibility, and irritability.

Clinical studies of an embodiment of the present invention yielded surprisingly effective and unanticipated results. The oral extended release dosage unit formulation of the present invention appeared to cause a sustained release of clonidine over a prolonged period of time. The side effects of transient sedation and rebound hyperarousal were overcome. Thus the present invention overcomes the "peak and trough" side effects of the traditional oral clonidine formulations to provide more stable therapeutic effects.

Of particular importance is the ease with which the capsule can be manufactured. It does not require elaborate and expensive equipment and can be prepared quickly and inexpensively in a local pharmacy from ordinarily available materials by any pharmacist properly instructed in its preparation.

These and other features, aspects, and advantages of the present invention will become better understood with reference to the following description and appended claims.

### BEST MODES FOR CARRYING OUT THE INVENTION

In accordance with the present invention, a formulation for the extended release of clonidine is provided. The extended release formulation of the present invention is an oral dosage unit comprising a homogenous mixture of clonidine or an analog thereof, one or more cellulose ethers, and one or more therapeutically inert, pharmaceutically accepted fillers, as defined in annexed claim 1.

Clonidine is a 9-carbon, two-ringed imidazoline derivative. As used herein, the term "clonidine" in its broodast sense denotes 2,6-dichloro-N-2-imidazolidinylidene benzeneamine, and benzeneamines structurally and functionally related thereto that are described in U.S. Pat. no. 3,454,701, to which reference is directed for its disclosure of such structurally and functionally related benzeneamines. With respect to the preferred embodiments of the present invention, the term "clonidine" denotes 2,6-dichloro-N-2-imidazolidinylidene benzeneamine.

Polymeric compositions have been widely used as a matrix for extended or sustained drug release formulations. For such applications, a highly hydrophilic polymeric composition is suitably employed. Cellulose ethers such as methyl cellulose and hydroxypropyl methylcellulose are among the polymeric compositions which have been most widely used in this manner. Other cellulose ethers, such as ethyl cellulose, methyl cellulose, hydroxypropyl cellulose, hydroxyethyl cellulose, sodium carboxymethyl cellulose, have also been used. They exhibit fast hydration forming a protective initial gel layer quickly through which the drug is released to the system. Once the initial gel layer is formed, it continues to allow additional water to penetrate into the mass. Soluble materials will wet, dissolve, and diffuse out of the matrix while insoluble materials will be held in place until the surrounding complex erodes or dissolves away. As the outer gel layer begins to fully hydrate and be dissolved, a new layer replaces it that is tight and strong enough to retard diffusion and sustain uniform drug release. Factors affecting the rate of hydration of the polymeric composition, and thereby the drug release rate, include its viscosity, concentration, particle size, and chemical makeup.

Another factor affecting the rate of gel formation or hydration of the polymeric composition used as an extended drug release matrix is the chemical characteristics of the drug employed. Certain polymers can be employed effectively for some drugs but not for others. The degree of water solubility of the drug, molecular weight of the drug, and the diffusion coefficient of the drug in hydrated polymer are critical.

The oral dosage unit of the embodiments of the present invention may also contain one or more compositions such as diluents or fillers which are therapeutically inert and pharmaceutically acceptable and provide bulk. Examples of such diluents or fillers include cornstarch, lactulose, and dextrose.

The oral dosage unit can be in the form of a tablet or a capsule. Tablets may be prepared or manufactured in accordance with an embodiment of the present invention on any conventional tableting equipment. Where the oral dosage unit is in the form of a capsule, the capsule may be any standard two-piece gelatin capsule of sufficient size for containing the formulation.

In the preparation of the oral extended release formulation of the present invention, clonidine tablets are ground into a fine powder and mixed with one or more cellulose ethers and one or more diluents or fillers and either tableted or inserted into a gelatin capsule. The amount of clonidine that is included per oral dosage unit may vary widely. The therapeutically effective dose range of 0.025 mg to 0.40 mg per unit is preferred to control most of the symptoms of the clinical disorders listed above which clonidine may benefit. The dose of the oral dosage unit can be exactly specified, however, as required.

The cellulose ethers or mixtures thereof employed as the extended release matrix in the present invention are ultrafine, rapidly hydrating formulations preferably having a number average molecular weight of at least 86,000 or a 2% aqueous solution of viscosity of at least 4000 cps and wherein at least 90% by weight of the cellulose ether particles can pass through a 100 mesh screen. An important aspect of the present invention is that the extended release profile of clonidine can be specified by the types or amounts of cellulose ethers used. The invention is thus very adaptable and versatile to each particular use. The oral dosage formulation herein described provides a preferred release period suitable for the dosing of clonidine twice per day, at twelve hour intervals.

A functionally effective amount of the cellulose ether composition is employed. Such an amount is an amount sufficient to extend the release of clonidine for up to twelve hours. Such an amount can vary and typically ranges from 30 to 70 weight percent, and preferably from 30 to 40 weight percent based on the weight of the capsule, although any functionally effective amount can be employed.

The preferred extended release matrix is hydroxypropyl methylcellulose such as Methocel®, which is manufactured by the Dow Chemical Company, U.S.A. The preferred Methocel® for an eight hour release period is E4M which has a hydroxypropoxyl substitution of from 7 to 12 weight percent, a methoxyl substitution of from 28 to 30 weight percent, a number average molecular weight of 86,000, a 2% aqueous solution of viscosity of 4000 cps and 95% by weight can pass through a 100 mesh screen. The preferred Methocel® for a twelve hour release period is K100M which has a hydroxypropoxyl substitution of from 7 to 12 weight percent, a methoxyl substitution of from 19 to 24 weight percent, a number average molecular weight of 246,000, a 2% aqueous solution of viscosity of 100,000 cps and at least 90% by weight can pass through a 100 mesh screen.

Diluents and fillers, such as cornstarch, lactulose, and dextrose, are included in the preparation of the present invention from 30 to 70 weight percent based on the weight of the capsule.

### INDUSTRIAL APPLICABILITY

The present invention should be used for the exact same indications as other forms of clonidine. The optimal dose is related to weight and age, as well as other factors such as the rate of biotransformation in the liver which can be quite variable among patients. Doses must therefore be individualized in each case by empiric trials. The best way to find the optimal dose of clonidine according to the embodiments of the present invention is to increase the dose slowly and to monitor the therapeutic effects and side effects. If the patient becomes sedated or paradoxically irritable with an increase in dose, then it is advisable to reduce the dose to that previously well-tolerated, hold at this dose for several weeks and then advance again as tolerated. To minimize sedation it is best to start with a very low dose, 0.025 mg. every twelve hours is preferred, and to work up at weekly intervals in increments of 0.025 mg. Clonidine tends to work better after longer use with additional benefits evident even after many months.

Clonidine has been found to be very useful in aiding the onset of sleep in Attention Deficit Hyperactivity Disorder patients. Such patients often have a lifetime history of difficulty getting to sleep. This difficulty with state regulation appears to be part of the Attention Deficit Hyperactivity Disorder complex of symptoms. The sedation experienced approximately one hour after the traditional oral tablet dose of clonidine which is troublesome in the day is often very useful at night in aiding the onset of sleep. One difficulty in a significant number of patients is the problem of waking about four to six hours later, sometimes with a nightmare. This rebound hyperarousal can limit the use of clonidine for this purpose.

As noted above, the present invention, with its slow release, has proven to be free of the problem of rebound hyperarousal. On the other hand, because of its slower onset of action it does not aid in the onset of sleep as effectively as the tablet form. One effective strategy is to give a formulation according to the present invention earlier in the evening, at 5 p.m., for example, to allow several hours for the onset of the effect. Another strategy which has proven very effective is to give a traditional oral tablet dose of clonidine, containing 0.05 mg. to 0.2 mg. clonidine, with the extended release formulation of the present invention containing 0.05 mg. to 0.2 mg. of clonidine. The tablet aids in the onset of sleep and the extended release form maintains an adequate level of medication throughout the night, thus preventing the "middle of the night" wakening and nightmare problems.

The following non-limiting example serves to further illustrate the invention. This example shows how to manufacture 100 units of an oral extended release formulation of clonidine in capsule form containing 0.025 mg. of clonidine in either eight or twelve hour release formulations.

Powdered clonidine is prepared by grinding twenty five 0.1 mg. clonidine tablets, then sifting the powder through a fine, 100 mesh screen to ensure small particle size. The powdered clonidine is mixed with 7 gm. of Methocel® K100M for 12 hour release or Methocel® E4M for 8 hour release. Cornstarch or lactulose is added until the total weight of the mixture equals 20 gm. The resulting mixture is homogenized by shaking and divided equally among 100 gelatin capsules each weighing 0.20 gm.

The above formulation was clinically tested in 50 patients in which clonidine had previously proved effective but side effects of sedation via the oral tablet route, or contact dermatitis via the transdermal patch, had limited its usefulness. By starting at doses at or below the tablet form, the risk of the drug not releasing at all resulting in no therapeutic effect, or the drug releasing too fast approximating the release of the tablet form, was minimized. optimal doses of clonidine ranged from 0.025 mg. to 0.15 mg. every 12 hours.

The results of the clinical tests yielded surprising and unanticipated results. The extended release capsule appeared as effective as the tablet or transdermal patch while producing significantly fewer side effects such as hyperarousal, contact dermatitis, and sedation. The twice or three times daily dosages proved more convenient for the patient and resulted in greater patient satisfaction and better compliance.

Although the present invention has been described in considerable detail with reference to certain preferred versions thereof, other versions are possible. For example, the extended release formulation of the present invention is effective when the drug to be delivered is methylphenidate or dextroamphetamine.

## Claims

1. An oral pharmaceutical dosage unit formulation for the extended release of clonidine or a clonidine analog to effect central alpha-adrenergic stimulation over a prolonged period upon administration thereof, wherein the oral dosage unit comprises:
a. 0.025 mg to 0.40 mg of a clonidine or clonidine analog of the formula 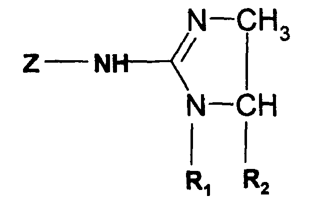 wherein R₁ and R₂ are each selected from the group consisting of hydrogen, methyl and ethyl, and Z is selected from the group consisting of:
2,6-dichlorophenyl,
2-trifluoromethyl-phenyl,
2-chloro-6-methyl-phenyl, 2-methyl-4-chloro-phenyl,
2-chloro-4-methyl-phenyl,
2-chloro-4-ethyl-phenyl,
2-chloro-6-ethyl-phenyl,
2-chloro-4-tert,butyl-phenyl,
2,6-dichloro-4-methyl-phenyl,
2,4-dichloro-6-methyl-phenyl,
2,4-dimethyl-6-chloro-phenyl, and
2,6-dimethyl-4-chloro-phenyl,
and its non-toxic pharmacologically acceptable acid addition salts;
as an homogenous mixture in
b. 30 to 70 percent by weight of one or more cellulose ethers, all having a number average molecular weight of greater than 30,000;
c. 30 to 70 percent by weight of therapeutically inert, pharmaceutically acceptable adjunct materials.

2. The extended release formulation according to claim 1, wherein the oral dosage unit is contained in a gelatin capsule.

3. The extended release formulation according to claim 1, wherein the oral dosage unit is in the form of a tablet.

4. The extended release formulation according to any preceding claim, wherein the amount of the one or more cellulose ethers in the oral dosage unit is 30 to 40 percent by weight.

5. The extended release formulation according to any preceding claim, wherein the cellulose ethers are selected from the group consisting of ethyl cellulose, methyl cellulose, hydroxypropyl cellulose, hydroxypropyl methylcellulose, hydroxyethyl cellulose, sodium carboxymethyl cellulose, and mixtures thereof.

6. The extended release formulation according to any preceding claim, wherein the one or more cellulose ethers have a number average molecular weight of at least 86,000.

7. The extended release formulation according to any preceding claim, wherein the one or more cellulose ethers have a viscosity of at least 4000 cps in 2% aqueous solution.

8. The extended release formulation according to any preceding claim, wherein the cellulose ether is hydroxypropyl methylcellulose.

9. The extended release formulation according to claim 8, wherein the hydroxypropyl methylcellulose has a hydroxypropoxyl substitution of from 7 to 12 weight percent, a methoxyl substitution of from 28 to 30 weight percent, a number average molecular weight of about 86,000, and a 2% aqueous solution viscosity of about 4000 cps.

10. The extended release formulation according to claim 8, wherein the hydroxypropyl methylcellulose has a hydroxypropoxyl substitution of from 7 to 12 weight percent, a methoxyl substitution of from 19 to 24 weight percent, a number average molecular weight of about 246,000, and a 2% aqueous solution viscosity of about 100,000 cps.

11. The extended release formulation according to any preceding claim, wherein the therapeutically inert, pharmaceutically acceptable adjunct material is selected from the group consisting of cornstarch, lactulose and dextrose.

12. The extended release formulation according to any preceding claim, wherein the release period is from 8 to 12 hours.

13. Use of
a. 0.025 mg to 0.40 mg of a clonidine or clonidine analog of the formula 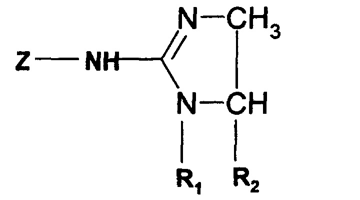 wherein R₁ and R₂ are each selected from the group consisting of hydrogen, methyl and ethyl, and Z is selected from the group consisting of:
2,6-dichlorophenyl,
2-trifluoromethyl-phenyl,
2-chloro-6-methyl-phenyl, 2-methyl-4-chloro-phenyl,
2-chloro-4-methyl-phenyl,
2-chloro-4-ethyl-phenyl,
2-chloro-6-ethyl-phenyl,
2-chloro-4-tert,butyl-phenyl,
2,6-dichloro-4-methyl-phenyl,
2,4-dichloro-6-methyl-phenyl,
2,4-dimethyl-6-chloro-phenyl, and
2,6-dimethyl-4-chloro-phenyl,
and its non-toxic pharmacologically acceptable acid addition salts;
as a homogenous mixture in
b. 30 to 70 percent by weight of one or more cellulose ethers, all having a number average molecular weight of greater than 30,000,
c. 30 to 70 percent by weight of therapeutically inert, pharmaceutically acceptable adjunct materials,
in the preparation of an extended release medicament
for the treatment of attention deficit hyperactivity disorder.

14. Use according to claim 13 in which the compound is clonidine (2,6-dichloro-N-2-imidazolidinglidenebenzeneamine).

15. Extended release formulation according to any one of claims 1 to 12 in which the compound is clonidine (2,6-dichloro-N-2-imidazolidinglidenebenzeneamine).

## Patentansprüche

1. Dosierungseinheit einer oralen pharmazeutischen Formulierung für die verzögerte Freisetzung von Clonidin oder eines Clonidinanalogons, um nach deren Verabreichung eine zentrale alpha-adrenerge Stimulation über eine längere Zeitspanne zu bewirken, worin die orale Dosierungseinheit umfasst:
a. 0,025 mg bis 0,40 mg Clonidin oder eines Clonidinanalogons mit der Formel 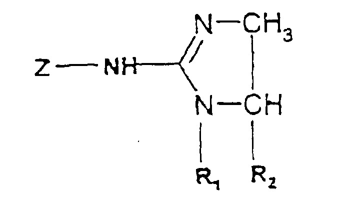 worin R₁ und R₂ jeweils unabhängig ausgewählt sind aus der Gruppe bestehend aus Wasserstoff, Methyl und Ethyl, und worin Z ausgewählt ist aus der Gruppe bestehend aus:
2,6-Dichlorphenyl,
2-Trifluormethylphenyl,
2-Chlor-6-methylphenyl,
2-Methyl-4-chlorphenyl,
2-Chlor-4-methylphenyl,
2-Chlor-4-ethylphenyl,
2-Chlor-6-ethylphenyl,
2-Chlor-4-tert-butylphenyl,
2,6-Dichlor-4-methylphenyl,
2,4-Dichlor-6-methylphenyl,
2,4-Dimethyl-6-chlorphenyl und
2,6-Dimethyl-4-chlorphenyl und deren nicht toxischen, pharmakologisch akzeptablen Säureadditionssalzen,
als ein homogenes Gemisch in
b. 30 bis 70 Gewichtsprozent von einem oder mehreren Celluloseethern, welche alle ein Zahlenmittel-Molekulargewicht von mehr als 30000 aufweisen;
c. 30 bis 70 Gewichtsprozent von therapeutisch inerten, pharmazeutisch akzeptablen Zusatzstoffen.

2. Formulierung mit verzögerter Freisetzung nach Anspruch 1, worin die orale Dosierungseinheit in einer Gelatinekapsel enthalten ist.

3. Formulierung mit verzögerter Freisetzung nach Anspruch 1, worin die orale Dosierungseinheit in Form einer Tablette vorliegt.

4. Formulierung mit verzögerter Freisetzung nach einem der vorhergehenden Ansprüche, worin die Menge des einen oder der mehreren Celluloseether in der oralen Dosierungseinheit 30 bis 40 Gewichtsprozent beträgt.

5. Formulierung mit verzögerter Freisetzung nach einem der vorhergehenden Ansprüche, worin die Celluloseether ausgewählt sind aus der Gruppe bestehend aus Ethylcellulose, Methylcellulose, Hydroxypropylcellulose, Hydroxypropylmethylcellulose, Hydroxyethylcellulose, Natriumcarboxymethylcellulose und Gemischen davon.

6. Formulierung mit verzögerter Freisetzung nach einem der vorhergehenden Ansprüche, worin der eine oder die mehreren Celluloseether ein Zahlenmittel-Molekulargewicht von mindestens 86000 aufweisen.

7. Formulierung mit verzögerter Freisetzung nach einem der vorhergehenden Ansprüche, worin der eine oder die mehreren Celluloseether in einer 2%-igen wässrigen Lösung eine Viskosität von mindestens 4000 cP aufweisen.

8. Formulierung mit verzögerter Freisetzung nach einem der vorhergehenden Ansprüche, worin der Celluloseether Hydroxypropylmethylcellulose ist.

9. Formulierung mit verzögerter Freisetzung nach Anspruch 8, worin die Hydroxypropylmethylcellulose eine Hydroxypropoxylsubstitution von 7 bis 12 Gewichtsprozent, eine Methoxylsubstitution von 28 bis 30 Gewichtsprozent, ein Zahlenmittel-Molekulargewicht von etwa 86000 und in einer 2%-igen wässrigen Lösung eine Viskosität von etwa 4000 cP aufweist.

10. Formulierung mit verzögerter Freisetzung nach Anspruch 8, worin die Hydroxypropylmethylcellulose eine Hydroxypropoxylsubstitution von 7 bis 12 Gewichtsprozent, eine Methoxylsubstitution von 19 bis 24 Gewichtsprozent, ein Zahlenmittel-Molekulargewicht von etwa 246000 und in einer 2%-igen wässrigen Lösung eine Viskosität von etwa 100000 cP aufweist.

11. Formulierung mit verzögerter Freisetzung nach einem der vorhergehenden Ansprüche, worin der therapeutisch inerte, pharmazeutisch akzeptable Zusatzstoff ausgewählt ist aus der Gruppe bestehend aus Maisstärke, Lactulose und Dextrose.

12. Formulierung mit verzögerter Freisetzung nach einem der vorhergehenden Ansprüche, worin die Zeitspanne der Freisetzung 8 bis 12 Stunden beträgt.

13. Verwendung von
a. 0,025 mg bis 0,40 mg Clonidin oder eines Clonidinanalogons mit der Formel 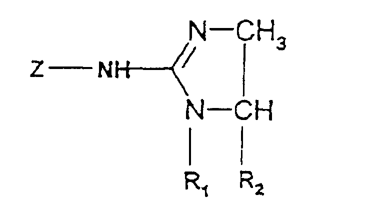 worin R₁ und R₂ jeweils unabhängig ausgewählt sind aus der Gruppe bestehend aus Wasserstoff, Methyl und Ethyl, und worin Z ausgewählt ist aus der Gruppe bestehend aus:
2,6-Dichlorphenyl,
2-Trifluormethylphenyl,
2-Chlor-6-methylphenyl,
2-Methyl-4-chlorphenyl,
2-Chlor-4-methylphenyl,
2-Chlor-4-ethylphenyl,
2-Chlor-6-ethylphenyl,
2-Chlor-4-tert-butylphenyl,
2,6-Dichlor-4-methylphenyl,
2,4-Dichlor-6-methylphenyl,
2,4-Dimethyl-6-chlorphenyl und
2,6-Dimethyl-4-chlorphenyl und deren nicht toxischen, pharmakologisch akzeptablen Säureadditionssalzen,
als ein homogenes Gemisch in
b. 30 bis 70 Gewichtsprozent von einem oder mehreren Celluloseethern, welche alle ein Zahlenmittel-Molekulargewicht von mehr als 30000 aufweisen;
c. 30 bis 70 Gewichtsprozent von therapeutisch inerten, pharmazeutisch akzeptablen Zusatzstoffen,
zur Herstellung eines Arzneimittels mit verzögerter Freisetzung zur Behandlung des hyperkinetischen Syndroms.

14. Verwendung nach Anspruch 13, worin die Verbindung Clonidin (2,6-Dichlor-N-2-imidazolidinglidenbenzolamin) ist.

15. Formulierung mit verzögerter Freisetzung nach einem der Ansprüche 1 bis 12, worin die Verbindung Clonidin (2,6-Dichlor-N-2-imidazolidinglidenbenzolamin) ist.

## Revendications

1. Formulation pharmaceutique d'unité posologique orale pour la libération prolongée de clonidine ou d'un analogue de clonidine pour effectuer une stimulation alpha-adrénergique centrale sur une période prolongée après l'administration de celle-ci, dans laquelle l'unité posologique orale comprend :
a. 0,025 mg à 0,40 mg de clonidine ou d'un analogue de clonidine de formule 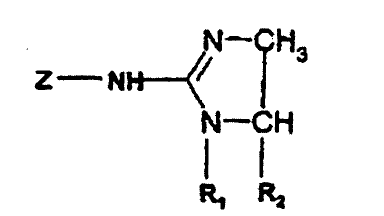 dans laquelle R₁ et R₂ sont chacun choisis dans le groupe constitué par l'hydrogène, un groupe méthyle et éthyle, et Z est choisi dans le groupe constitué par :
un groupe 2,6-dichlorophényle,
un groupe 2-trifluorométhyl-phényle,
un groupe 2-chloro-6-méthyl-phényle, 2-méthyl-4-chloro-phényle,
un groupe 2-chloro-4-méthyl-phényle,
un groupe 2-chloro-4-éthyl-phényle,
un groupe 2-chloro-6-éthyl-phényle,
un groupe 2-chloro-4-tert,butyl-phényle,
un groupe 2,6-dichloro-4-méthyl-phényle,
un groupe 2,4-dichloro-6-méthyl-phényle,
un groupe 2,4-diméthyl-6-chloro-phényle, et
un groupe 2,6-diméthyl-4-chloro-phényle,
et ses sels d'addition avec un acide non toxiques, pharmacologiquement acceptables ;
sous la forme d'un mélange homogène dans
b. 30 à 70 pour cent en poids d'un ou plusieurs éthers de cellulose, tous ayant un poids moléculaire moyen en nombre supérieur à 30 000 ;
c. 30 à 70 pour cent en poids de matières adjuvantes thérapeutiquement inertes, pharmaceutiquement acceptables.

2. Formulation à libération prolongée selon la revendication 1, dans laquelle l'unité posologique orale est contenue dans une capsule de gélatine.

3. Formulation à libération prolongée selon la revendication 1, dans laquelle l'unité posologique orale est sous la forme d'un comprimé.

4. Formulation à libération prolongée selon l'une quelconque des revendications précédentes, dans laquelle la quantité du ou des éthers de cellulose dans l'unité posologique orale est de 30 à 40 pour cent en poids.

5. Formulation à libération prolongée selon l'une quelconque des revendications précédentes, dans laquelle les éthers de cellulose sont choisis dans le groupe constitué par l'éthylcellulose, la méthylcellulose, l'hydroxypropylcellulose, l'hydroxypropylméthylcellulose, l'hydroxyéthylcellulose, la carboxyméthylcellulose sodique et des mélanges de celles-ci.

6. Formulation à libération prolongée selon l'une quelconque des revendications précédentes, dans laquelle le ou les éthers de cellulose ont un poids moléculaire moyen en nombre d'au moins 86 000.

7. Formulation à libération prolongée selon l'une quelconque des revendications précédentes, dans laquelle le ou les éthers de cellulose ont une viscosité d'au moins 4000 cP dans une solution aqueuse à 2 %.

8. Formulation à libération prolongée selon l'une quelconque des revendications précédentes, dans laquelle l'éther de cellulose est l'hydroxypropylméthylcellulose.

9. Formulation à libération prolongée selon la revendication 8, dans laquelle l'hydroxypropylméthylcellulose a une substitution hydroxypropoxyle de 7 à 12 pour cent en poids, une substitution méthoxyle de 28 à 30 pour cent en poids, un poids moléculaire moyen en nombre d'environ 86 000 et une viscosité dans une solution aqueuse à 2 % d'environ 4000 cP.

10. Formulation à libération prolongée selon la revendication 8, dans laquelle l'hydroxypropylméthylcellulose a une substitution hydroxypropoxyle de 7 à 12 pour cent en poids, une substitution méthoxyle de 19 à 24 pour cent en poids, un poids moléculaire moyen en nombre d'environ 246 000 et une viscosité dans une solution aqueuse à 2 % d'environ 100 000 cP.

11. Formulation à libération prolongée selon l'une quelconque des revendications précédentes, dans laquelle la matière adjuvante thérapeutiquement inerte, pharmaceutiquement acceptable est choisie dans le groupe constitué par l'amidon de maïs, le lactulose et le dextrose.

12. Formulation à libération prolongée selon l'une quelconque des revendications précédentes, dans laquelle la période de libération est de 8 à 12 heures.

13. Utilisation de
a. 0,025 mg à 0,40 mg de clonidine ou d'un analogue de clonidine de formule 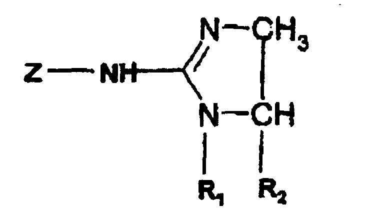 dans laquelle R₁ et R₂ sont chacun choisis dans le groupe constitué par l'hydrogène, un groupe méthyle et éthyle, et Z est choisi dans le groupe constitué par :
un groupe 2,6-dichlorophényle,
un groupe 2-trifluorométhyl-phényle,
un groupe 2-chloro-6-méthyl-phényle, 2-méthyl-4-chloro-phényle,
un groupe 2-chloro-4-méthyl-phényle,
un groupe 2-chloro-4-éthyl-phényle,
un groupe 2-chloro-6-éthyl-phényle,
un groupe 2-chloro-4-tert,butyl-phényle,
un groupe 2,6-dichloro-4-méthyl-phényle,
un groupe 2,4-dichloro-6-méthyl-phényle,
un groupe 2,4-diméthyl-6-chloro-phényle, et
un groupe 2,6-diméthyl-4-chloro-phényle,
et ses sels d'addition avec un acide non toxiques, pharmacologiquement acceptables ;
sous la forme d'un mélange homogène dans
b. 30 à 70 pour cent en poids d'un ou plusieurs éthers de cellulose, tous ayant un poids moléculaire moyen en nombre supérieur à 30 000 ;
c. 30 à 70 pour cent en poids de matières adjuvantes thérapeutiquement inertes, pharmaceutiquement acceptables,
dans la préparation d'un médicament à libération prolongée pour le traitement du trouble d'hyperactivité avec déficit de l'attention.

14. Utilisation selon la revendication 13, dans laquelle le composé est la clonidine (2,6-dichloro-N-2-imidazolidinglidènebenzèneamine).

15. Formulation à libération prolongée selon l'une quelconque des revendications 1 à 12, dans laquelle le composé est la clonidine (2,6-dichloro-N-2-imidazolidinglidènebenzèneamine).
